Europäisches Patentamt

European Patent Office

Office européen des brevets

(19)

(11) Publication number: **0 306 192 B1**

(12) **EUROPEAN PATENT SPECIFICATION**

(45) Date of publication of patent specification: **27.05.92**

(51) Int. Cl.5: **A61K 47/02**, A61K 31/44, A61K 31/50, A61K 31/505, A61K 47/10, A61K 47/22

(21) Application number: **88307752.1**

(22) Date of filing: **22.08.88**

(54) **Permeation enhancement compositions.**

(30) Priority: **28.08.87 US 90726**

(43) Date of publication of application:
**08.03.89 Bulletin 89/10**

(45) Publication of the grant of the patent:
**27.05.92 Bulletin 92/22**

(84) Designated Contracting States:
**AT BE CH DE ES FR GB GR IT LI LU NL SE**

(56) References cited:
EP-A- 0 129 284          EP-A- 0 161 918
EP-A- 0 245 126          EP-A- 0 271 983
GB-A- 2 014 134          US-A- 4 405 616
US-A- 4 501 980

CHEMICAL ABSTRACTS, vol. 98, no. 13, 28th March 1983, page 43, abstract no. 100968p, Columbus, Ohio, US; E. MIKKELSEN et al.: "Comparison of the effects of a new vasodilator pinacidil and nifedipine on isolated blood vessels", & ACTA PHARMACOL. TOXICOL. 1982, 51(5),407-12

(73) Proprietor: **ELI LILLY AND COMPANY**
**Lilly Corporate Center**
**Indianapolis Indiana 46285(US)**

(72) Inventor: **Valia, Kirti Himatlal**
**7970 Springwater Drive, West**
**Indianapolis Indiana 46256(US)**

(74) Representative: **Tapping, Kenneth George et al**
**Erl Wood Manor**
**Windlesham Surrey, GU20 6PH(GB)**

Rank Xerox (UK) Business Services

INTERNATIONAL JOURNAL OF PHARMACEU-
TICS, vol. 33, no. 1-3, 1986, pages 37-43,
Elsevier Science Publishers B.V., Amster-
dam, NL; E. TOUITOU: "Transdermal delivery
of anxiolytics; in vitro skin permeation of
midazolam maleate and diazepam"

PATENT ABSTRACTS OF JAPAN, vol. 10, no.
185 (C-357)[2241], 27th June 1986; & JP-A-61
33 128 (NITTO ELECTRIC IND. CO. LTD)
17-02-1986

International Journal of Pharmaceutics, Vol.
33, 1986 p. 37-43

**Description**

This invention relates to improved pharmaceutical formulations containing pharmacologically active ingredients.

Percutaneous or transdermal delivery of pharmacologically active agents has become feasible in recent years largely due to vehicles therefor which allow increased permeation of said agents into the body surface to which applied. Certain advantages are attendant to percutaneous drug delivery such as avoidance of the varied absorption and metabolism which can be encountered by oral therapy, avoidance of first pass hepatic metabolism and improved patient compliance as a result of a simplified therapeutic regimen. In many cases, drugs which would appear to be ideal candidates for percutaneous delivery are found to have such low permeability that they cannot be delivered at therapeutically effective rates. In attempts to obviate this problem, various materials and compositions have been suggested as permeation enhancers to effect the transdermal delivery of the pharmacologically active agent. Among these are the following: dimethyl sulfoxide (U.S. Patent 3,551,554); various 1-substituted azacycloalkan-2-ones including 1-dodecylazacycloheptan-2-one referred to hereinafter as azone (U.S. Patents 4,562,075, 4,405,616, 4,326,893 and 3,989,816); sugar esters in combination with sulfoxide or phosphine oxide (U.S. Patents 4,130,667, 4,130,643, 4,046,886, 3,952,099 and 3,896,238); lower alkyl amides (U.S. Patent 3,472,931); certain aliphatic sulfoxides (U.S. Patent 3,903,256); a composition containing glycerol monooleate, ethanol and isopropyl myristate (U.S. Patent 4,335,115); a binary mixture of 1-dodecylazacycloheptan-2-one and a compound selected from a diol or a second N-substituted azacycloalkyl-2-one (U.S. Patent 4,557,934); and polyethylene glycol monolaurate (U.S. Patent 4,568,343). The use of Azone® in permeation enhancement compositions is described in EP-A-271983, EP-A-245126 and International Journal of Pharmaceutics, Vol. 33 (1986), 37-43

According to the present invention, there is provided a permeation enhancement composition for providing increased percutaneous absorption of a pharmacologically active agent comprising from 30 to 70 parts by weight ethanol, from 0.1 to 10 parts by weight azone, from 30 to 80 parts by weight water, and optionally, from 10 to 30 parts by weight propylene glycol.

The permeation enhancement compositions of the present invention effect the increased percutaneous absorption of the pharmacologically active agents disposed therein to an extent greater than that observed if any one component of said compositions were used alone as the permeation enhancement means for said agent. Without being bound by theory, it would appear that each of the ethanol, water and, when present, propylene glycol act individually as solvents for the pharmacologically active agents, the permeation of each of which is enhanced by the action of azone®. Additionally, the ethanol and propylene glycol exhibit some permeation enhancement characteristics which, when taken in the aggregate with their solvent effects, renders compositions with azone® which show exquisite permeation enhancement capacity.

The permeation enhancement compositions of the present invention utilize, in addition to ethanol, water and propylene glycol (if present), the known permeation enhancer Azone®. As noted above, chemically azone is 1-dodecylazacycloheptan-2-one and is prepared as described in U.S. Patent 4,316,893. The present permeation enhancement compositions contain from 30 to 70 parts by weight ethanol (preferably 200 proof), from 0.1 to 10 parts by weight azone®, and from 30 to about 80 parts by weight water. Optionally, from 10 to 30 parts by weight of propylene glycol may be added to the formulation. A preferred composition which contains propylene glycol is a composition containing from 30 to 50 parts by weight ethanol, from 0.1 to 10 parts by weight azone, from 30 to 50 parts by weight water and from 10 to 30 parts by weight propylene glycol. A particularly preferred composition which contains propylene glycol is a composition having 37 parts by weight ethanol, 3 parts by weight azone, 40 parts by weight water and 20 parts by weight propylene glycol. A preferred composition which does not contain propylene glycol is one containing from 35 to 70 parts by weight ethanol, from 0.1 to 10 parts by weight azone® and from 35 to 80 parts by weight water. A particularly preferred composition which does not contain propylene glycol is one containing 52 parts by weight ethanol, 3 parts by weight azone® and 45 parts by weight water. Formulation of these compositions may be achieved by conventional methods, as by the simple mixing of all components thoroughly. The above-noted ranges set forth for each component of the permeation enhancement compositions of the present invention are critical for achieving the unexpected increase in percutaneous absorption of pharmacologically active agents as illustrated hereinafter. However, the skilled artisan will readily appreciate that compositions containing diols other than propylene glycol and alcohols other than ethanol (such as 2-propanol) may find utility in permeation enhancement compositions as a component of the formulation.

The permeation enhancement compositions of the present invention are utilized to increase the percutaneous absorption of a pharmacologically active agent disposed therewith. "Increased percutaneous absorption" refers to absorption of a pharmacologically active agent to an extent greater than if said agent were not disposed with a permeation enhancement composition of the present invention. As used herein, the term "pharmacologically active agent" means those compounds described in Examples 1 to 4, infra.

It is contemplated that the permeation enhancement compositions of the present invention will find utility in both humans and animals, i.e., will have both medical and veterinary applications for providing increased percutaneous absorption of pharmacologically active agents. It must be pointed out that as used herein, the term "percutaneous" refers not only to the passage of such agents through skin (typically intact) but also to the passage of pharmacologically active agents through membranous tissue such as mucosal membranes. For these purposes, various pharmaceutical dosage forms are available for percutaneous administration and are well known in the art and include, for example, creams, lotions, gels, ointments, suppositories, sprays, aerosols and any of a wide variety of transdermal devices for use in the relatively continuous administration of pharmacologically active agents as described in any of the following: U.S. Patent Nos. 3,598,122, 3,598,123, 3,710,795, 3,731,683, 3,742,951, 3,814,097, 3,921,636, 3,972,995, 3,993,072, 3,993,073, 3,996,934, 4,031,894, 4,060,084, 4,069,307, 4,077,407, 4,201,211, 4,230,105, 4,292,299 and 4,292,303. Preferred pharmaceutical dosage forms are transdermal devices. A particularly preferred transdermal device is as disclosed in U.S. Patent 3,710,795. Of course, as other such dosage forms are developed the permeation enhancement compositions of the present invention shall also find applicability. It will be readily evident to the skilled artisan that the dosage form utilized may also incorporate certain pharmaceutically-acceptable excipients which are conventional in the art. These include, for example, gelling agents, cream and ointment bases.

One or more pharmacologically active agents are present in the pharmaceutical dosage form in an effective amount, i.e., an amount calculated to achieve and maintain blood levels which will bring about the desired beneficial or therapeutic effect over the period of time desired. These amounts will vary depending upon the relative potency of the pharmacologically active agent utilized, the amount of such agent required for the desired beneficial or therapeutic effect, the physiological half-life of the agent, the specific formulation of the permeation enhancement composition used. Suffice it to say that conventional dose titration techniques, which are well within the skill of the artisan, may be utilized to determine the amount of the pharmacologically active agent present in the ultimate pharmaceutical dosage form for any specific situation encountered.

The pharmacologically active agent is administered by known techniques such as by placing a source (i.e., a pharmaceutical dosage form) containing said agent and permeation enhancement composition therefor (including any excipients if needed) on a body surface and maintaining said source on said body surface in agent and composition transmitting relation thereto. Alternatively, the permeation enhancement compositions of the present invention may be used separately from the pharmaceutical dosage form containing the pharmacologically active agent. For example, the body surface to which the pharmacologically active agent is to be applied may be pretreated with a permeation enhancement composition of the present invention prior to application of the pharmaceutical dosage form containing said agent.

In order to further illustrate the present invention, various of the permeation enhancement compositions described herein were formulated with pharmacologically active agents. These formulations were then tested for their capacity to permeate human cadaver skin using the apparatus and method described by Valia et al. in Drug Development and Industrial Pharmacy, 10(7), 951-981 (1984). Briefly, the apparatus used was a skin permeation cell consisting of two cylindrical half-cells in mirror image. Each of the half-cells was composed of a solution compartment which was enclosed inside of a water jacket compartment. Each of the half-cells was equipped with a closable sampling port and a depression in the solution compartment which serves as the platform for a star-head rotating magnet. The magnets stirred the solution in the half-cells at a constant rate of 600 revolutions per minute by an external synchronous driving unit. Both donor and receptor compartments were thermostatically controlled at a constant temperature by circulating 37°C water through the water jacket compartment by means of an external circulator. The skin sample (human cadaver skin) used in the investigations was then mounted between the two half-cells. In the donor compartment was placed the formulation containing the pharmacologically active agent (i.e., the agent and permeation enhancement composition) and in the receptor compartment was placed the same formulation absent the pharmacologically active agent. During the diffusion period (usually from 0 to 48 hours) samples were withdrawn periodically from the receptor compartment and subjected to HPLC analysis for a determination of the concentration of the pharmacologically active agent therein.

Example 1

Table I depicts the results of a permeation study which was conducted as described above for the free base of a dihydropyridine calcium channel antagonist formulated, as by thorough mixing, with various permeation enhancement compositions of the present invention. This dihydropyridine (referred to in Table I as "test compound") is a potent coronary and peripheral vasodilator and is chemically (+)-1,4-dihydro-2,6-dimethyl-4-(3-nitrophenyl)-3,5-pyridinedicarboxylic acid, methyl 1-(phenylmethyl)-3-pyrrolidinyl ester. In Table I (and subsequent tables) all amounts shown are in parts by weight unless specifically denoted otherwise therein.

TABLE I

| Test Compound[a] | Ethanol | Azone® | Water | PG[b] | Flux[c] |
|---|---|---|---|---|---|
| 1.0 | 62 | 3 | 35 | -- | 0.648 |
| 0.5 | 54 | 1 | 45 | -- | 0.417 |
| 0.5 | 52 | 3 | 45 | -- | 0.897 |
| 1.0 | 37 | 3 | 40 | 20 | 22.621 |
| 1.0 | 40 | 3 | 37 | 20 | 11.068 |
| 1.0 | 52 | 3 | 45 | -- | 2.115 |
| 1.0 | 52 | 3 | 45 | -- | 1.427 |
| 1.0 | 52 | 3 | 45 | -- | 4.780 |
| 0.5 | 37 | 3 | 40 | 20 | 8.249 |
| 0.5 | 37 | 3 | 40 | 20 | 8.956 |
| 0.5 | 37 | 3 | 40 | 20 | 8.101 |

[a] The amount of the test compound present in the donor compartment of the skin permeation cell at the start of the investigation
[b] Propylene glycol
[c] Refers to the steady state flux (rate of permeation) of the test compound through the skin sample in micrograms per square centimeter per hour ($\mu$g/cm$^2$/hour)

The data shown in Table I clearly demonstrate the permeation enhancement effects of the present compositions. In contrast, when the test compound of Example 1 (20 parts by weight) was formulated in azone alone, a steady state flux of only 0.037 $\mu$g/cm$^2$/hour was attained. Similarly, when the test compound of Example 1 (2.5 parts by weight) was formulated with ethanol alone a flux of only 0.118 $\mu$g/cm$^2$/hour was attained although this value increased to 0.361 $\mu$g/cm$^2$/hour when the amount of the test compound was increased to 4.5 parts by weight in a formulation with ethanol alone. A formulation of the test compound of Example 1 (8 parts by weight) in polyethylene glycol 400 alone, or in a mixture of polyethylene glycol 400 (90 parts by weight) and ethanol (10 parts by weight) achieved a negligible flux of only 0.0002 $\mu$g/cm$^2$/hour. Thus, a 5 to 100 fold increase in permeation of human cadaver skin was obtained when the test compound of Example I was formulated with a permeation enhancement composition of the present invention.

Example 2

A second pharmacologically active agent was assessed in the skin permeation cell using the methods described above. This test compound possesses both positive inotropic and vasodilator activities and is chemically 1,3-dihydro-3,3-dimethyl-5-(1,4,5,6-tetrahydro-6-oxo-3-pyridazinyl)-2H-indol-2-one The results of this study are shown in Table II.

TABLE II

| Test Compound[a] | Ethanol | Azone® | Water | PG[b] | Flux[c] |
|---|---|---|---|---|---|
| 0.3[d] | 37 | 3 | 40 | 20 | 13.80 |
| 0.3[d] | 52 | 3 | 45 | -- | 6.75 |

[a,b,c] As defined in Table 1
[d] Formulated with 2 parts by weight hydroxypropyl cellulose as a gelling agent

Example 3

Table III shows the results of a permeation study using the methods described above for a test compound known as pinacidil (i.e., (±)-N-cyano-N'-4-pyridinyl-N''(1,2,2-trimethylpropyl)guanidine, monohydrate. Pinacidil is pharmacologically active as an antihypertensive agent.

TABLE III

| Test Compound[a] | Ethanol | Azone® | Water | PG[b] | Flux[c] |
|---|---|---|---|---|---|
| 2[d] | 37 | 3 | 40 | 20 | 21.40 |
| 2[d] | 52 | 3 | 45 | -- | 31.780 |

[a,b,c] As defined in Table I
[d] Formulated with 2 parts by weight hydroxypropyl cellulose as a gelling agent

Example 4

A fourth pharmacologically active agent was assessed in the skin permeation cell using the methods described above. This test compound is the free base of a dopamine agonist and is chemically trans(-)5,5A,6,-7,8,9,9A,10-octahydro-6-propylpyrimidino(4,5-G)quinolin-2-amine. The results of this study are shown in Table IV.

TABLE IV

| Test Compound[a] | Ethanol | Azone® | Water | PG[b] | Flux[c] |
|---|---|---|---|---|---|
| 1.0 | 52 | 3 | 45 | -- | 78.946 |
| 1.0 | 37 | 3 | 40 | 20 | 80.251 |

[a,b,c] As defined in Table I

**Claims**
**Claims for the following Contracting States : AT, BE, CH, DE, FR, GB, IT, LI, LU, NL, SE**

1.  A permeation enhancement composition for providing increased percutaneous absorption of a pharmacologically active agent selected from ( + )-1,4-dihydro-2,6-dimethyl-4-(3-nitrophenyl)-3,5-pyridinedicarboxylic acid, methyl 1-(phenylmethyl)-3-pyrrolidinyl ester; 1,3-dihydro-3,3-dimethyl-5-(1,4,5,6-tetrahydro-6-oxo-3-pyridazinyl)-2H-indol-2-one; (±)-N-cyano-N'-4-pyridinyl-N''-(1,2,2-trimethylpropyl)guanidine, monohydrate; and trans()5,5A,6,7,-8,9,9A,10-octahydro-6-propylpyrimidino(4,5-G)-quinoline-2-amine; comprising from 30 to 70 parts by weight ethanol, from 0.1 to 10 parts by weight 1-dodecylazacycloheptan-2-one, from 30 to 80 parts by weight water.

2.  The composition of Claim 1 further comprising from 10 to 30 parts by weight of propylene glycol.

3.  The composition of claim 2 containing from 30 to 50 parts by weight ethanol, from 0.1 to 10 parts by weight 1-dodecylazacycloheptan-2-one, from 30 to 50 parts by weight water and from 10 to 30 parts by weight propylene glycol.

4.  The composition of claim 1 containing 15 from 35 to 70 parts by weight ethanol, from 0.1 to 10 parts by weight 1-dodecylazacycloheptan-2-one and from 35 to 80 parts by weight water.

5.  The composition of claim 3 containing 37 parts by weight ethanol, 3 parts by weight 1-dodecylazacycloheptan-2-one, 40 parts by weight water and 20 parts by weight propylene glycol.

6. The composition of claim 4 containing 52 parts by weight ethanol, 3 parts by weight 1-dodecylazacycloheptan-2-one and 45 parts by weight water.

7. The composition of claim 5 or 6 present in a pharmaceutical dosage form and additionally containing an effective amount of a pharmacologically active agent and pharmaceutically-acceptable excipients.

**Claims for the following Contracting States : ES, GR**

1. A process for the preparation of a permeation enhancement composition for providing increased percutaneous absorption of a pharmacologically active agent selected from ( + )-1,4-dihydro-2,6-dimethyl-4-(3-nitrophenyl)-3,5-pyridinedicarboxylic acid, methyl 1-(phenylmethyl)-3-pyrrolidinyl ester; 1,3-dihydro-3,3-dimethyl-5-(1,4,5,6-tetrahydro-6-oxo-3-pyridazinyl)-2H-indol-2-one; (±)-N-cyano-N'-4-pyridinyl-N''-(1,2,2-trimethylpropyl)guanidine, monohydrate; and trans(-)5,5A,6,7,8,9,9A,10-octahydro-6-propylpyrimidino(4,5-G)quinoline-2-amine; comprising admixing from 30 to 70 parts by weight ethanol, from 0.1 to 10 parts by weight 1-dodecylazacycloheptan-2-one, and from 30 to 80 parts by weight water.

2. The process of claim 1 further comprising the addition of from 10 to 30 parts by weight polypropylene glycol

3. The process of claim 2 wherein the composition contains from 30 to 50 parts by weight ethanol, from 0.1 to 10 parts by weight 1-dodecylazacycloheptan-2-one, from 30 to 50 parts by weight water and from 10 to 30 parts by weight propylene glycol.

4. The process of claim 1 wherein the composition contains from 35 to 70 parts by weight ethanol, from 0.1 to 10 parts by weight 1-dodecylazacycloheptan-2-one and from 35 to 80 parts by weight water.

5. The process of claim 3 wherein the composition contains 37 parts by weight ethanol, 3 parts by weight 1-dodecylazacycloheptan-2-one, 40 parts by weight water and 20 parts by weight propylene glycol.

6. The process of claim 4 wherein the composition contains 52 parts by weight ethanol, 3 parts by weight 1-dodecylazacycloheptan-2-one and 45 parts by weight water.

7. The process of claim 5 or 6 wherein the composition is present in a pharmaceutical dosage form and additionally containing an effective amount of a pharmacologically active agent and pharmaceutically-acceptable excipients.

**Revendications**
**Revendications pour les Etats contractants suivants : AT, BE, CH, DE, FR, GB, IT, LI, LU, NL, SE**

1. Composition améliorant la pénétration pour procurer une augmentation quant à l'absorption par voie percutanée d'un agent pharmacologiquement actif choisi parmi l'ester méthyl-1-(phénylméthyl)-3-pyrrolidinylique de l'acide ( + )-1,4-dihydro-2,6-diméthyl-4-(3-nitrophényl)-3,5-pyridine-dicarboxylique; 1a 1,3-dihydro-3,3-diméthyl-5(1,4,5,6-tétrahydro-6-oxo-3-pyridazinyl)-2H-indol-2-one; le monohydrate de la (±)-N-cyano-N'-4-pyridinyl-N''-(1,2,2-triméthylpropyl)-guanidine et la trans-(-)-5,5a,6,7,8,9,9a,10-octahydro-6-propylpyrimidino(4,5-G)quinoléin-2-amine; comprenant de 30 à 70 parties en poids d'éthanol, de 0,1 à 10 parties en poids de 1-dodécylazacycloheptan-2-one, de 30 à 80 parties en poids d'eau.

2. Composition selon la revendication 1 comprenant, en outre, de 10 à 30 parties en poids de propylèneglycol.

3. Composition selon la revendication 2 contenant de 30 à 50 parties en poids d'éthanol, de 0,1 à 10 parties en poids de 1-dodécylazacycloheptan-2-one, de 30 à 50 parties en poids d'eau et de 10 à 30 parties en poids de propylèneglycol.

4. Composition selon la revendication 1 contenant de 35 à 70 parties en poids d'éthanol, de 0,1 à 10 parties en poids de 1-dodécylazacycloheptan-2-one et de 35 à 80 parties en poids d'eau.

**5.** Composition selon la revendication 3 contenant 37 parties en poids d'éthanol, 3 parties en poids de 1-dodécylazacycloheptan-2-one, 40 parties en poids d'eau et 20 parties en poids de propylèneglycol.

**6.** Composition selon la revendication 4 contenant 52 parties en poids d'éthanol, 3 parties en poids de 1-dodécylazacycloheptan-2-one et 45 parties en poids d'eau.

**7.** Composition selon la revendication 5 ou 6 présente dans une forme posologique de produit pharmaceutique et contenant, en outre, une quantité efficace d'un agent pharmacologiquement actif, ainsi que des excipients pharmaceutiquement acceptables.

**Revendications pour les Etats contractants suivants : ES, GR**

**1.** Procédé pour la préparation d'une composition améliorant la pénétration pour procurer une augmentation quant à l'absorption par voie percutanée d'un agent pharmacologiquement actif choisi parmi l'ester méthyl-1-(phénylméthyl)-3-pyrrolidinylique de l'acide ( + )-1,4-dihydro-2,6-diméthyl-4-(3-nitrophényl)-3,5-pyridine-dicarboxylique; la 1,3-dihydro-3,3-diméthyl-5-(1,4,5,6-tétrahydro-6-oxo-3-pyridazinyl)-2H-indol-2-one; le monohydrate de la (±)-N-cyano-N'-4-pyridinyl-N''-(1,2,2-triméthylpropyl)-guanidine et la trans-(-)-5,5a,6,7,8,9,9a,10-octahydro-6-propylpyrimidino(4,5-G)quinoléin-2-amine; comprenant de 30 à 70 parties en poids d'éthanol, de 0,1 à 10 parties en poids de 1-dodécylazacycloheptan-2-one, et de 30 à 80 parties en poids d'eau.

**2.** Procédé selon la revendication 1 comprenant, en outre, l'addition de 10 à 30 parties en poids de propylèneglycol.

**3.** Procédé selon la revendication 2 dans lequel la composition contient de 30 à 50 parties en poids d'éthanol, de 0,1 à 10 parties en poids de 1-dodécylazacycloheptan-2-one, de 30 à 50 parties en poids d'eau et de 10 à 30 parties en poids de propylèneglycol.

**4.** Procédé selon la revendication 1 dans lequel la composition contient de 35 à 70 parties en poids d'éthanol, de 0,1 à 10 parties en poids de 1-dodécylazacycloheptan-2-one et de 35 à 80 parties en poids d'eau.

**5.** Procédé selon la revendication 3 dans lequel la composition contient 37 parties en poids d'éthanol, 3 parties en poids de 1-dodécylazacycloheptan-2-one, 40 parties en poids d'eau et 20 parties en poids de propylèneglycol.

**6.** Procédé selon la revendication 4 dans lequel la composition contient 52 parties en poids d'éthanol, 3 parties en poids de 1-dodécylazacycloheptan-2-one et 45 parties en poids d'eau.

**7.** Procédé selon la revendication 5 ou 6 dans lequel la composition est présente dans une forme posologique de produit pharmaceutique et contient, en outre, une quantité efficace d'un agent pharmacologiquement actif, ainsi que des excipients pharmaceutiquement acceptables.

**Patentansprüche**

**Patentansprüche für folgende Vertragsstaaten : AT, BE, CH, DE, FR, GB, IT, LI, LU, NL, SE**

**1.** Zusammensetzung zur Verbesserung der Permeation, um eine erhöhte perkutane Absorption eines pharmakologisch aktiven Mittels, ausgewählt aus ( + )-1,4-Dihydro-2,6-dimethyl-4-(3-nitro-phenyl)-3,5-pyridindicarbonsäure-methyl-1-(phenylmethyl)-3-pyrrolidinylester; 1,3-Dihydro-3,3-dimethyl-5-(1,4,5,6-tetrahydro-6-oxo-3-pyridazinyl)-2H-indol-2-on; (±)-N-Cyano-N'-4-pyridinyl-N''(1,2,2-trimethylpropyl)-guanidinmonohydrat; und trans(-)5,5A,6,7,8,9,9A,10-Octahydro-6-propylpyrimidino(4,5-G)chinolin-2-amin, zu liefern, umfassend 30 bis 70 Gew.-Teile Ethanol, 0,1 bis 10 Gew.-Teile 1-Dodecylazacycloheptan-2-on, 30 bis 80 Gew.-Teile Wasser.

**2.** Zusammensetzung nach Anspruch 1, weiter umfassend 10 bis 30 Gew.-Teile Propylenglykol.

**3.** Zusammensetzung nach Anspruch 2, enthaltend 30 bis 50 Gew.-Teile Ethanol, 0,1 bis 10 Gew.-Teile 1-Dodecylazacycloheptan-2-on, 30 bis 50 Gew.-Teile Wasser und 10 bis 30 Gew.-Teile Propylengylkol.

**4.** Zusammensetzung nach Anspruch 1, enthaltend 35 bis 70 Gew.-Teile Ethanol, 0,1 bis 10 Gew.-Teile 1-Dodecylazacycloheptan-2-on und 35 bis 80 Gew.-Teile Wasser.

**5.** Zusammensetzung nach Anspruch 3, enthaltend 37 Gew.-Teile Ethanol, 3 Gew.-Teile 1-Dodecylazacycloheptan-2-on, 40 Gew.-Teile Wasser und 20 Gew.-Teile Propylenglykol.

**6.** Zusammensetzung nach Anspruch 4, enthaltend 52 Gew.-Teile Ethanol, 3 Gew.-Teile 1-Dodecylazacycloheptan-2-on und 45 Gew.-Teile Wasser.

**7.** Zusammensetzung nach Anspruch 5 oder 6, enthalten in einer pharmazeutischen Dosierungsform und zusätzlich enthaltend eine wirksame Menge eines pharmakologisch aktiven Mittels und pharmazeutisch annehmbarer Hilfsstoffe.

**Patentansprüche für folgende Vertragsstaaten : ES, GR**

**1.** Verfahren zur Herstellung einer Zusammensetzung zur Verbesserung der Permeation, um eine erhöhte perkutane Absorption eines pharmakologisch aktiven Mittels, ausgewählt aus ( + )-1,4-Dihydro-2,6-dimethyl-4-(3-nitrophenyl)-3,5-pyridindicarbonsäure-methyl-1-(phenylmethyl)-3-pyrrolidinylester; 1,3-Dihydro-3,3-dimethyl-5-(1,4,5,6-tetrahydro-6-oxo-3-pyridazinyl)-2H-indol-2-on; (±)-N-Cyano-N'-4-pyridinyl-N''-(1,2,2-trimethylpropyl)guanidinmonohydrat; und trans(-)5,5A,6,7,8,9,9A,10-Octahydro-6-propylpyrimidino(4,5-G)chinolin-2-amin, zu liefern, umfassend, daß man 30 bis 70 Gew.-Teile Ethanol, 0,1 bis 10 Gew.-Teile 1-Dodecylazacycloheptan-2-on und 30 bis 80 Gew.-Teile Wasser vermischt.

**2.** Verfahren nach Anspruch 1, weiter umfassend die Zugabe von 10 bis 30 Gew.-Teilen Propylenglykol.

**3.** Verfahren nach Anspruch 2, worin die Zusammensetzung 30 bis 50 Gew.-Teile Ethanol, 0,1 bis 10 Gew.-Teile 1-Dodecylazacycloheptan-2-on, 30 bis 50 Gew.-Teile Wasser und 10 bis 30 Gew.-Teile Propylengylkol enthält.

**4.** Verfahren nach Anspruch 1, worin die Zusammensetzung 35 bis 70 Gew.-Teile Ethanol, 0,1 bis 10 Gew.-Teile 1-Dodecylazacycloheptan-2-on und 35 bis 80 Gew.-Teile Wasser enthält.

**5.** Verfahren nach Anspruch 3, worin die Zusammensetzung 37 Gew.-Teile Ethanol, 3 Gew.-Teile 1-Dodecylazacycloheptan-2-on, 40 Gew.-Teile Wasser und 20 Gew.-Teile Propylenglykol enthält.

**6.** Verfahren nach Anspruch 4, worin die Zusammensetzung 52 Gew.-Teile Ethanol, 3 Gew.-Teile 1-Dodecylazacycloheptan-2-on und 45 Gew.-Teile Wasser enthält.

**7.** Verfahren nach Anspruch 5 oder 6, worin die Zusammensetzung in einer pharmazeutischen Dosierungsform enthalten ist und zusätzlich eine wirksame Menge eines pharmakologisch aktiven Mittels und pharmazeutisch annembarer Hilfsstoffe enthält.